**Europäisches Patentamt**

**European Patent Office**

**Office européen des brevets**

(19)

(11) Numéro de publication : **0 059 949**
**B2**

(12)

# NOUVEAU FASCICULE DE BREVET EUROPÉEN

(45) Date de publication du nouveau fascicule du brevet :
09.11.88

(21) Numéro de dépôt : 82101708.4

(22) Date de dépôt : 05.03.82

(51) Int. Cl.⁴ : **B 01 J 13/02, B 41 M 5/00**

(54) **Procédé d'obtention de microcapsules isodiamétrales à diamètre réglé.**

(30) Priorité : 09.03.81 FR 8104800

(43) Date de publication de la demande :
15.09.82 Bulletin 82/37

(45) Mention de la délivrance du brevet :
01.08.84 Bulletin 84/31

(45) Mention de la décision concernant l'opposition :
09.11.88 Bulletin 88/45

(84) Etats contractants désignés :
**AT BE DE GB IT SE**

(56) Documents cités :
**EP-A- 0 026 914**
**DE-A- 2 119 933**
**DE-A- 2 832 637**
**FR-A- 1 334 124**
**FR-A- 2 167 021**
**FR-A- 2 413 123**
**US-A- 4 187 194**
**SCIENCE PROGRES DECOUVERTE, vol. 99, février**
**1971 , Société d'Editions Scientifiques, Paris (FR) M.**
**MESLE et al.: "Les microcapsules font la publicité**
**qui sent", pages 14-23**

(73) Titulaire : **S.E.P.C. S.A. Société D'EXPLOITATION DES**
**PROCEDES COATEX**
**35, Cours Aristide Briand**
**F-69300 Caluire (FR)**

(72) Inventeur : **Bousquet, Hubert**
**35 cours Aristide-Briand**
**F-69300 Caluire (FR)**
Inventeur : **Egraz, Jean-Bernard**
**Impasse Moulin Carron**
**F-69130 Ecully (FR)**
Inventeur : **Ravet, Georges**
**La Guigonnière St. Genis les Ollières**
**F-69190 Craponne (FR)**

(74) Mandataire : **Eggert, Hans-Gunther, Dr.**
**Räderscheidtstrasse 1**
**D-5000 Köln 41 (DE)**

EP 0 059 949 B2

## Description

L'invention concerne un procédé perfectionné d'encapsulation de substances finement dispersées dans une phase liquide conduisant à la formation de microcapsules isodiamétrales.

L'invention concerne enfin l'encapsulation des substances très diverses, telles que les encres, les colorants, les réactifs chimiques divers, les produits pharmaceutiques, les produits phytosanitaires, les produits ignifuges, etc.

La technique de micro-encapsulation qui permet d'enfermer dans une enveloppe de très faible dimension des substances solides, liquides ou volatiles, et généralement réactives à l'égard de leur environnement, est déjà bien connue.

Le grand nombre de documents publiés dans la littérature spécialisée en ce domaine reflète la nécessité pour l'homme de l'art de conserver des substances solides, liquides ou volatiles dans des microréceptacles protecteurs de telle manière qu'elles soient toujours disponibles au moment choisi pour leur exploitation, c'est-à-dire qu'elles gardent l'intégralité de leurs propriétés jusqu'à l'instant de leur libération par éclatement ou par biodégradation de leur enveloppe.

De nombreuses substances ont déjà fait l'objet d'encapsulation, parmi lesquelles figurent des agents aromatiques, tels que les parfums, des colorants, des réactifs chimiques dont les colorants réactifs, des colles et adhésifs, des agents phytosanitaires tels certains agents biocides, des engrais, etc.

Mais il faut également observer que de nombreuses publications décrivent l'utilisation de microcapsules dans le domaine des matériaux de reproduction par pression, disposant d'un support en papier, tels que les documents simples ou multiples connus sous les appellations chimiques autonomes et chimiques contacts.

Les préparations de microcapsules formées à partir de matières polymères naturelles ou synthétiques ont été décrites dans de multiples publications, qui sont les conséquences de certaines observations de l'homme de l'art sur les avantages qu'elles procurent.

Un premier procédé de préparation des parois des microcapsules, qui est l'un des plus anciens et des plus usités, pratique la coacervation à partir d'une solution aqueuse de colloïdes hydrophiles, d'origine naturelle, tels que les gélatines, gomme arabique, mais aussi d'origine synthétique, tels que les copolymères d'éther polyvinylméthylique et d'acide maléique, et les carboxyméthylcelluloses.

Un tel procédé ne pouvant se pratiquer qu'avec des concentrations faibles en colloïdes, l'encapsulation des substances à enrober ne peut se réaliser que dans un milieu aqueux à faible teneur en ces substances, de l'ordre de 18 à 23 %, rendant particulièrement onéreux ce procédé d'encapsulation.

Plus que ce premier inconvénient, un autre inconvénient peut se manifester d'une manière plus gênante encore. Car il est connu de l'homme de l'art que de nombreux colloïdes hydrophiles, d'origine naturelle ou dérivés de produits naturels comme la carboxyméthylcellulose, peuvent être l'objet d'une destruction par des micro-organismes, rendant poreuse la paroi des capsules produites et interdisant, par exemple, toute conservation prolongée de substances enrobées, sensibles à l'atmosphère ambiante. Ainsi, les substances encapsulées risquent de s'oxyder, de s'échapper de leur réceptacle, faisant douter de l'intérêt qu'il y a à réaliser l'encapsulation de certaines substances, en particulier quand celles-ci sont volatiles.

En raison de ces premiers inconvénients, l'homme de l'art s'est attaché à trouver des procédés améliorés de préparation des parois de microcapsules utilisant des matières d'enrobage, non plus d'origine naturelle, mais d'origine synthétique, qui, après polymérisation, restent insensibles aux agressions des micro-organismes, aux agents chimiques, ces matières étant en outre moins onéreuses que les colloïdes hydrophiles précités. En d'autres termes, ces procédés améliorés se proposent de produire des parois de microcapsules qui ne laissent plus échapper tout ou partie des substances enrobées, et qui soient moins fragiles à la pression ou au choc que les parois antérieurement réalisées à partir de colloïdes hydrophyles.

C'est pourquoi ont été proposés des procédés de formation des parois de microcapsules par polymérisation in situ, c'est-à-dire dans le milieu où se trouvent les substances à enrober.

Un premier procédé très simple de fabrication par polymérisation in situ de microcapsules à parois synthétiques consiste à disperser la substance à enrober (qui est liquide ou rendue liquide par solubilisation dans un solvant) au sein d'une solution aqueuse contenant un précondensat soluble d'urée/formaldéhyde, puis à provoquer une polymérisation par condensation et, enfin, à préciter le polymère formé par addition d'un acide.

Comme l'ont montré les multiples expériences de fabrication des parois de microcapsules, la dispersion de la substance à enrober au sein de la solution aqueuse du précondensat d'urée/formaldéhyde doit être soumise à une agitation permanente, pendant que s'opère la polymérisation par condensation. Car, en l'absence d'une telle agitation, la dispersion devient instable, et l'on constate alors que les gouttelettes les plus fines de la substance à enrober se regroupent par un phénomène de coalescence, rendant pratiquement impossible le réglage de leur diamètre dont les dimensions deviennent très irrégulières, en allant du microscopique au macroscopique. Dès lors, il en est de même après l'encapsulation de ces gouttelettes, qui donne des produits encapsulés de dimension éminemment variable.

2

Or, il serait souhaitable qu'un tel procédé d'encapsulation conduise, d'une part, à la formation de produits encapsulés de dimension régulière et, d'autre part, à la reproduction de ces dimensions, d'une opération de production à une autre. Un tel procédé amélioré a été décrit dans le brevet français n° 2 332 798 qui vise la fabrication de microcapsules par enrobage de matières particulaires finement divisées au moyen d'enveloppes en matière polymère et qui consiste à préparer une dispersion de ces matières particulaires dans un milieu aqueux contenant en solution un précondensat d'urée/formaldéhyde, puis à y introduire un mélange, soluble dans l'eau, d'un précondensat de mélamine/formaldéhyde et d'un polymère comportant de préférence des groupes alcool, amine, amide, acide ou dérivé d'acide, susceptibles d'être réticulés par ce précondensat, enfin à provoquer la réticulation, par une catalyse acide, des enveloppes de polymère formées autour des matières particulaires.

Malgré les améliorations apportées par ce procédé aux divers inconvénients formulés précédemment, il subsiste malgré tout des difficultés résultant d'une certaine hétérogénéité de la dispersion, préjudiciable à la régularité du diamètre des microcapsules produites. L'instabilité de cette dispersion pourrait être réduite par l'usage d'agents tensio-actifs, comme cela a été proposé et décrit, par exemple, dans le brevet français n° 2 093 646. Ce document propose un procédé d'encapsulation de substances finement dispersées dans un liquide à l'aide d'un polymère surfactif-réactif, qui comporte d'abord la dispersion de ladite substance dans un agent dispersant en présence du polymère surfactif-réactif, capable de former un composé insoluble dans ledit agent dispersant, puis l'insolubilisation du polymère surfactif-réactif, en provoquant la formation d'une suspension primaire de capsules, enfin l'ajout d'une solution d'un précondensat aminoplaste faiblement tensio-actif dans la première suspension, entraînant la formation d'une suspension secondaire de capsules à parois renforcées.

Un tel procédé découle naturellement de l'observation des inconvénients décelés dans les publications antérieures. La première observation résulte de la nécessité de disperser finement les substances solides ou liquides à enrober dans la solution d'un condensat soluble au moyen d'une agitation mécanique appropriée, en présence du polymère surfactif-réactif, pendant que s'effectue la formation de l'enveloppe de manière que les particules dispersées le restent ou que les particules agglomérées soient à nouveau séparées les unes des autres avant d'être enrobées. Il est en effet notoirement connu que la solution du condensat soluble n'a pas la propriété de favoriser la fine dispersion des substances à enrober, et conduit à la fabrication de capsules plus ou moins grossières, présentant, en tout cas, une grande disparité dans leurs diamètres. La deuxième observation procède du fait que l'emploi d'une agitation mécanique adéquate pour créer et maintenir l'état de dispersion appropriée au sein de la solution du condensat soluble, au moment de l'encapsulation, ralentit la formation de l'enveloppe polymère, régulière et épaisse, qui n'offre plus une résistance mécanique suffisante pour supporter sans dommage, c'est-à-dire sans éclatement, une telle agitation.

Ainsi, l'art antérieur conduit à cette première conclusion, qu'il n'est pas possible de fabriquer des microcapsules de faible diamètre, mais surtout de diamètre régulier, quels que soient les moyens mis en œuvre dans les procédés employés. Car, pour obtenir une dispersion fine et assez régulière des substances à encapsuler, il faut soumettre le milieu de formation desdites capsules à une intense agitation, qui provoque l'éclatement d'au moins une fraction des microcapsules formées. En conséquence, le milieu de formation desdites capsules soumises à agitation doit l'être en présence d'au moins un agent protecteur qui, atténuant l'effet de dispersion, conduit à la réalisation de microcapsules grossières et irrégulières.

Un autre procédé de fabrication par polymérisation in situ de microcapsules à parois synthétiques a été proposé, qui consiste, selon une méthode de polymérisation interfaciale, à mettre en contact une phase contenant la substance à encapsuler et un réactif de polycondensation avec une autre phase non miscible à la première, qui contient un second réactif susceptible de réagir avec le premier en donnant un polycondensat. Or, comme la réaction entre les deux composés de produit, lors de la mise en contact des deux phases, à l'interface des gouttelettes et du milieu de dispersion, dès que l'enveloppe, même très mince, est formée autour de la gouttelette, les deux composés sont séparés l'un de l'autre, empêchant la réaction de se poursuivre, et donnant naissance à des microcapsules à parois fragiles.

C'est en raison de cet inconvénient attaché à la polymérisation interfaciale qu'un procédé amélioré d'encapsulation a été proposé dans le brevet français n° 2 413 123, qui préconise, selon un procédé en deux étapes, d'abord la dispersion dans une phase aqueuse d'une phase organique contenant simultanément la dispersion hydrophobe liquide à encapsuler et au moins un réactif hydrophobe polyfonctionnel muni d'au moins un groupe carbonyle ou sulfonyle, puis à provoquer la polycondensation du réactif hydrophobe avec au moins une amine polyfonctionnelle, initialement inhibée par salification, mais dont les fonctions amines sont libérées par ajout d'un agent alcalin de force supérieure à celle de ladite amine, provoquant ainsi le déclenchement de la polycondensation. Mais, comme la réaction se produit pratiquement au moment même où la dispersion de la phase organique est réalisée au sein de la phase aqueuse contenant le réactif hydrophile, ce procédé conduit à la production de microcapsules de diamètres très dispersés se situant entre 1 et 100 μ.

Ainsi, l'examen des diverses publications traitant des procédés d'encapsulation de substances actives diverses, au moyen de matières polymères, révèle l'existence de nombreuses difficultés conduisant à des résultats souvent décevants, voire même médiocres, en raison des multiples inconvénients précédemment énumérés, tels que fragilité des parois des microcapsules, dispersion

3

0 059 949

difficile des matières à enrober, polymérisation in situ des matières polymères constituant les parois, trop rapidement arrêtée par les phénomènes d'interface au sein des dispersions, enfin production de microcapsules à dimensions très irrégulières.

Or, si l'usage de la microcapsule est ou peut être répandu dans de très nombreux domaines d'applications, comme le laisse pressentir la littérature spécialisée, l'un des domaines où la microcapsule est particulièrement employée est celui des matériaux de reproduction par pression. Tel est, par exemple, le cas du matériau appelé papier report qui comporte en liasse une feuille supérieure, dont le verso est revêtu de microcapsules contenant une solution d'agents chromogènes, puis des feuilles intermédiaires dont les rectos sont recouverts d'un agent auxochrome, tel que par exemple une argile acide, une résine phénolique, etc., pouvant réagir avec la solution chromogène, tandis que leurs versos sont revêtus, comme la première feuille, de microcapsules contenant lesdits agents chromogènes. La pression exercée sur les feuilles, par dactylographie par exemple, provoque la rupture des microcapsules, et l'agent chromogène ainsi libéré réagit avec l'agent auxochrome qui le révèle par l'apparition d'une couleur.

Mais, comme la littérature spécialisée l'a abondamment décrit, les microcapsules peuvent contenir également des encres, des colorants, des réactifs chimiques divers, des produits pharmaceutiques, des agents d'aromatisation, des pesticides, des herbicides, etc., c'est-à-dire tout corps que l'on peut mettre en solution, en suspension dans une phase liquide, ou se trouvant lui-même dans cet état, et qui est enfermé dans une microcapsule à enveloppe polymère. Cette microcapsule doit conserver son contenu jusqu'à sa libération volontaire par rupture, fusion, dissolution de sa paroi ou encore, dans certains cas particuliers, par diffusion à travers la paroi de ladite capsule.

La libération du contenu de la microcapsule qui répond à un acte volontaire ne doit pas être provoquée d'une manière accidentelle par rupture prématurée de sa paroi. Tel est pourtant le cas décrit dans la littérature spécialisée, qui concerne les microcapsules destinées à des applications de reproduction graphique par pression, dont il est dit que les procédés connus pratiqués pour leur fabrication conduisent à des microcapsules à parois trop fragiles, se rompant, par exemple, lors de l'enduction des surfaces de papier selon les techniques d'imprimerie telles que l'offset, l'héliogravure et la typographie.

Comme les microcapsules produites selon ces procédés le sont avec des diamètres très hétérogènes, les risques de rupture accidentelle par pression des microcapsules déposées sur une feuille discontinue ou non sont multipliés par la présence de capsules de grande dimension.

C'est pourquoi l'homme de l'art, n'ayant pu apporter une solution raisonnable au problème soulevé par l'hétérogénéité des diamètres des microcapsules produites selon l'art antérieur, s'est attaché à les protéger, lors de leur emploi, contre toute rupture accidentelle et libération prématurée de la substance incluse, au moyen de matériaux particuliers, tels que par exemple des grains d'amidon, d'un diamètre supérieur à celui de la microcapsule la plus grande.

Pour accroître la résistance à la rupture accidentelle des microcapsules déposées sur un support papier par exemple destiné à la reprographie, il a été proposé de revêtir ledit support, en mélange avec les microcapsules, d'une matière protectrice à base de fibres cellulosiques et de grains d'amidon, qui devrait laisser toute latitude de rupture volontaire des microcapsules par pression (par exemple par dactylographie), mais qui les protégeraient contre les actions mécaniques accidentelles, se produisant lors de frottements ou de stockages.

Le principe édicté par cette protection consiste à appliquer, sur le support choisi, les microcapsules et les matériaux protecteurs et à les lier au moyen d'une solution gélifiée d'amidon. Un tel procédé est décrit par exemple dans le brevet français n° 2 170 687.

De la DE-A-2 119 933, on connaît un procédé de préparation de microcapsules, pour lequel la substance à enrober aussi bien que la matière formant la paroi se trouvent en une seule phase organique. La matière de l'enveloppe de la capsule se dépose de la phase dispersée à l'interface par évaporation du solvant. Comme solvant pour la substance à encapsuler ou celle formant la paroi, on utilise en particulier le chloroforme ou le chlorure de méthylène. Pour la préparation de l'émulsion de la phase organique dans l'eau, on met en œuvre un dispositif, avec lequel il est possible de produire une forte action mécanique de cisaillements.

Dans la DE-A-2 832 637, on décrit un procédé de fabrication de microcapsules, pour lequel sont polymérisés de l'urée et du formaldéhyde en présence d'un polyélectrolyte anionique et d'un sel d'ammonium et se forme une membrane constituant la paroi à partir d'une résine formaldéhyde-urée autour de gouttelettes d'un liquide oléagineux hydrophobe. Pour cela, peu importe manifestement la densité du solvant organique pour la substance à enrober les conditions de mélange et de température lors de la formation de l'émulsion huile dans l'eau.

Ainsi, l'examen de l'art antérieur révèle que les procédés de micro-encapsulation connus jusqu'à ce jour conduisent à l'obtention de microcapsules irrégulières dont les parois sont, par voie de conséquence, plus ou moins fragiles.

Poursuivant ses recherches en ce domaine, la titulaire a étudié et mis au point un procédé d'encapsulation de substances diverses finement dispersées dans une phase aqueuse, conduisant à l'obtention de microcapsules isodiamétrales, de diamètre réglé, contenant la substance à encapsuler.

Le procédé de micro-encapsulation de substances diverses dans le but d'obtenir des microcapsules simultanément isodiamétrales et de diamètre réglé, selon l'invention consiste

4

à mettre en présence une solution aqueuse de polymères et/ou copolymères hydrosolubles comportant au moins l'une des fonctions chimiques appartenant au groupe constitué par les radicaux —COOH, —OH, —CONH₂, —CONHR, —CONRR', —NH₂, —NHR et NRR', constituant la matière de l'enveloppe-réceptacle de la capsule, et une solution des substances à enrober dans au moins un solvant organique hydrophobe, de densité proche de 1, du groupe des solvants polyaryles,

à amener le milieu liquide ainsi constitué à une température choisie entre 15 et 85 °C, de préférence entre 35 et 60 °C,

à le soumettre pendant un temps compris dans l'intervalle de 1 à 60 min, de préférence de 20 à 35 min à une action mécanique de cisaillement correspondant à un coefficient de cisaillement compris entre 8 000 et 70 000 s⁻¹, de préférence compris entre 15 000 et 45 000 s⁻¹ en le maintenant à cette température choisie pendant l'action de cisaillement,

à régler le pH de la suspension des microcapsules isodiamétrales dans le milieu aqueux résultant de cette action dans l'intervalle de 3,5 à 7,5,

à introduire un agent de réticulation dans la suspension précitée, et

à abaisser la température de la suspension précitée de préférence jusqu'à la température ambiante.

Selon l'invention, l'action de cisaillement pratiquée sur le milieu liquide formé par la mise en présence et la dispersion de solvant porteur de la substance à enrober au sein du solvant porteur de la matière enrobante se définit par un coefficient de cisaillement qui s'exprime généralement par la relation :

$$C = \frac{\emptyset \times 3,14 \times N}{60 \times e}$$

relation dans laquelle :

— $\emptyset$ est le diamètre, exprimé en mètres, d'un rotor, tel que par exemple une turbine,

— N est le nombre de tours réalisés par le rotor pendant l'unité de temps exprimée en minutes, et

— e est l'entrefer, exprimé en mètres, mesurant la distance existant entre le rotor et un stator constitué par une couronne fixe entourant le rotor.

La titulaire a constaté que le coefficient de cisaillement jouait un rôle important dans la réalisation des microcapsules isodiamétrales.

Si le coefficient de cisaillement est trop faible, c'est-à-dire inférieur à 8 000 s⁻¹, les microcapsules obtenues ne sont pas isodiamétrales, et leur diamètre offre une dispersion très importante qui, pour 95 % d'entre elles, peut se situer par exemple entre 1 et 50 μ. En outre, les capsules ainsi obtenues possèdent une enveloppe d'épaisseur irrégulière, créant pour les plus grosses une fragilité accrue des parois.

Il semble, comme a pu le constater la titulaire, que le coefficient de cisaillement ne puisse pas dépasser 70 000 s⁻¹, valeur pour laquelle il a été constaté un taux non négligeable de destruction des microcapsules formées.

C'est pourquoi le coefficient de cisaillement est de préférence choisi entre 15 000 et 45 000 s⁻¹.

La solution aqueuse des polymères et/ou copolymères générateurs de l'enveloppe de la capsule est, en général, préparée par dissolution à chaud de ces matières, selon une température comprise entre 10 et 100 °C, et de préférence entre 30 et 60 °C.

De même, la solution de la substance à enrober dans un solvant organique hydrophobe, ou dans un mélange de solvants organiques hydrophobes, est obtenue, en général, par dissolution à chaud de ladite substance, à une température comprise entre 10 et 150 °C, et de préférence comprise entre 80 et 110 °C.

Le mélange constitué par la solution aqueuse de polymère et/ou copolymères générateurs de l'enveloppe de la capsule et par la solution dans le solvant organique hydrophobe de la substance à encapsuler est porté à une température au moins égale à celle de l'ambiance, température choisie habituellement entre 15 et 85 °C, mais de préférence entre 35 et 60 °C.

Puis ledit mélange est alors soumis à l'action de cisaillement correspondant au choix du coefficient de cisaillement dans l'intervalle précédemment énuméré, par tout moyen connu de l'homme de l'art.

Mais la titulaire a également observé, grâce aux nombreuses expérimentations qu'elle a réalisées pour étudier le procédé selon l'invention, qu'il était possible non seulement de produire des microcapsules isodiamétrales, mais encore d'en régler volontairement le diamètre moyen, grâce à un choix judicieux et précis, dans l'intervalle précité, de la température du mélange. Elle a en effet constaté que, selon le choix de la température à laquelle est porté le mélange de la solution aqueuse de polymères et/ou copolymères générateurs de l'enveloppe de la capsule et de la solution hydrophobe de la substance à encapsuler, l'action mécanique de cisaillement pratiquée selon une valeur de coefficient choisi dans l'intervalle de 8 000 à 70 000 s⁻¹ provoque la formation de microcapsules dont le diamètre moyen diminue quand la température du mélange augmente.

C'est ainsi que le diamètre moyen des microcapsules peut être diminué, par exemple, dans un rapport de 5 à 1 quand la température du mélange des solutions, soumis à l'action mécanique de cisaillement, évolue dans un rapport de 1 à 1,5.

Le temps pendant lequel le mélange constitué par la solution aqueuse de polymère et/ou copolymères générateurs de l'enveloppe de la capsule et par la solution dans le solvant organique hydrophobe de la substance à encapsuler est soumis à l'action de cisaillement selon l'invention est normalement compris entre 1 et 60 min, mais de préférence entre 20 et 35 min.

Dès lors que les paramètres précédemment énumérés sont choisis dans les intervalles antérieurement précisés, le volume total du mélange constitué par la solution aqueuse des polymères et/ou copolymères générateurs de l'enveloppe de la capsule et par la solution organique hydrophobe de la substance à enrober, qui est soumis à l'action de cisaillement selon l'invention, doit subir un nombre de passages, par recyclage, dans la zone de cisaillement. On a pu établir que le nombre souhaitable de recyclages dudit volume total dans la zone de cisaillement devait être compris entre 10 et 200, mais de préférence entre 12 et 55.

La concentration, exprimée en pour-cent en poids des polymères et/ou copolymères de la solution aqueuse génératrice de l'enveloppe de la capsule, est en général entre 2 et 15 %, mais de préférence entre 3 et 8 %.

De même, la concentration en pour-cent en poids, au sein du solvant organique hydrophobe de la substance solubilisée à encapsuler, est en général comprise entre 20 et 2 %, et de préférence entre 10 et 5 %, hormis le cas où la substance à encapsuler est le solvant organique hydrophobe lui-même, représentant dans ce cas 100 % de substance à encapsuler.

Selon le procédé de l'invention, la solution aqueuse contenant les polymères et/ou copolymères générateurs de l'enveloppe de la capsule est mise en présence de la solution organique hydrophobe contenant la substance à enrober en une quantité telle que le rapport massique des polymères et/ou copolymères hydrosolubles et de la substance à enrober soit compris entre 0,05 et 0,3.

La titulaire a en effet vérifié, comme le laissait pressentir l'art antérieur, que, si ce rapport est inférieur à 0,05, il est pratiquement impossible d'effectuer l'encapsulation, par défaut de polymères et/ou de copolymères, tandis que, si ce rapport est supérieur à 0,3, il se produit une augmentation importante de la viscosité du milieu, empêchant l'action mécanique de cisaillement de se produire par la forte diminution de la transmission de l'énergie correspondante et conduisant ainsi à une sous-production de microcapsules. C'est pourquoi le rapport massique des polymères et/ou copolymères hydrosolubles et de la substance à enrober est fixé, de préférence, dans l'intervalle de 0,07 à 0,15.

Après qu'a été effectuée l'opération de cisaillement, c'est-à-dire après qu'a été achevée la micro-encapsulation isodiamétrale de la substance à enrober, il importe que le pH de la suspension des microcapsules dans le milieu aqueux résultant du traitement soit amené à un optimum pour assurer la réticulation d'un précondensat, ajouté à ladite suspension, dans le but d'insolubiliser et de consolider les parois des microcapsules formées. Ce pH dépend de la nature des polymères et/ou copolymères choisis pour former lesdites parois, mais aussi de celle des précondensats introduits pour réaliser la réticulation. Ainsi, on peut aisément déterminer par expérimentation les conditions de pH propres à chaque cas particulier.

Et la titulaire, par ce moyen, a pu établir que l'intervalle de pH souhaitable se situe, en général, entre les limites de 3,5 à 7,5, mais de préférence entre 4,8 et 6,5.

L'acide servant à ajuster le pH n'est pas critique et peut être choisi, par exemple, dans le groupe des acides organiques tels que l'acide formique, l'acide acétique, l'acide oxalique, mais aussi dans celui des acides forts minéraux, tels que l'acide chlorhydrique.

Toutefois, si les polymères et/ou copolymères solubles dans l'eau, choisis pour constituer la paroi des microcapsules, présentent une acidité naturelle, la quantité d'acide nécessaire pour ajuster le pH peut être moindre et éventuellement nulle.

Le précondensat introduit en tant qu'agent de réticulation, permettant ainsi d'insolubiliser les microcapsules produites, est choisi parmi ceux bien connus de l'homme de l'art, tels que par exemple le formol, le formaldéhyde, les mélamines/formaldéhydes, les urées/formaldéhydes, le glyoxal, le glutaraldéhyde.

Il peut être introduit en une seule fois ou encore par petites fractions en un certain temps, par exemple en 30 min.

Enfin, la suspension des microcapsules isodiamétrales ainsi insolubilisées est refroidie, le refroidissement amenant de préférence la température du milieu à la température ambiante.

Dès lors que la réticulation des matières d'enrobage est achevée et que la température du milieu a été abaissée au niveau souhaité, on peut élever le pH de la suspension des microcapsules isodiamétrales jusqu'à le rendre alcalin pour empêcher toute réaction ultérieure desdites matières, par introduction d'une solution aqueuse d'hydroxyde ou d'amines solubles dans l'eau.

En pratique, le procédé selon l'invention d'encapsulation de substances finement dispersées dans une phase liquide par formation de microcapsules isodiamétrales comporte les étapes suivantes :

a) Préparation d'une solution aqueuse des polymères et/ou copolymères hydrosolubles comportant au moins l'une des fonctions OH, COOH, CO—NH$_2$, CONHR, CO—NRR', NH$_2$, NHR, NRR', destinée à former l'enveloppe des microcapsules, par dissolution, à une température comprise entre 10 et 100 °C, de 2 à 15 % en poids desdits polymères et/ou copolymères.

b) Préparation d'une solution de la substance à enrober dans au moins un solvant organique hydrophobe par dissolution, à une température comprise entre 10 et 150 °C, de 20 à 2 % en poids de ladite substance, quand ladite substance n'est pas le solvant organique hydrophobe lui-même.

c) Mélange, en l'absence de toute agitation, de la solution aqueuse de polymères et/ou copolymères hydrosolubles, générateurs de l'enveloppe des microcapsules isodiamétrales, avec le solvant organique

6

seul ou contenant en solution la substance à enrober, selon des quantités telles que le rapport massique des polymères et/ou copolymères hydrosolubles et de la substance à enrober soit compris entre 0,05 et 0,3.

d) Réglage de la température du mélange précédent jusqu'à l'obtention de la température choisie pour produire les microcapsules isodiamétrales au diamètre souhaité.

e) Soumission du mélange précédent, se trouvant porté à la température choisie, à une action de cisaillement correspondant à un coefficient d'au moins 8 000 s$^{-1}$.

f) Maintien du mélange précédent sous l'action de cisaillement pendant un temps de 1 à 60 min, de telle manière que le volume total dudit mélange subisse un nombre de passages par recyclage dans la zone de cisaillement compris entre 10 et 200.

g) Dès la fin de l'action mécanique de cisaillement, réglage du pH de la suspension des microcapsules isodiamétrales dans le milieu aqueux résultant de cette action, dans l'intervalle de 3,5 à 7,5.

h) Introduction dans la suspension précitée d'un agent de réticulation tel qu'un précondensat de mélamine/formaldéhyde, urée/formaldéhyde, glyoxal, dans le but d'insolubiliser les parois des microcapsules et d'en renforcer leur résistance mécanique.

i) Abaissement de la température de la suspension aqueuse des microcapsules de préférence jusqu'à la température ambiante.

j) Eventuellement, élévation du pH de la suspension aqueuse des microcapsules jusqu'à le rendre alcalin.

Les polymères et/ou copolymères hydrosolubles, générateurs des parois des microcapsules isodiamétrales, obtenues selon le procédé de l'invention, disposant au moins de l'une des fonctions hydroxyles (—OH), amine primaire (—NH$_2$), secondaire (—NHR) ou tertiaire (—NRR'), amide primaire (—CONH$_2$), secondaire (—CONHR) ou tertiaire (—CONRR') dans lesquels les radicaux R et R' sont des alkyles, des aryles ou des alkylaryles, acide (—COOH) ou dérivé d'acide.

Des exemples de polymères et/ou copolymères hydrosolubles préférés peuvent être cités, tels que les polymères et/ou copolymères dérivés de la cellulose, par exemple l'hydroxypropylcellulose, la méthylhydroxypropylcellulose, les carboxyméthylcelluloses ; les protéines et leurs dérivés, tels que les gélatines, par exemple l'agar-agar, la gomme arabique ; les polymères et/ou copolymères à base de mélamine et de formaldéhyde, ou d'urée et de formaldéhyde ; les polyacides ; les polyesters ; les copolymères d'anhydrides, par exemple, d'éther polyvinylméthylique et d'anhydride maléique ou de polyéthylène et d'anhydride maléique ; les polyacrylamides ou les copolymères d'acrylamide, par exemple les copolymères d'acrylamide et d'acide acrylique.

Le choix des polymères et/ou copolymères hydrosolubles destinés à former la substance enrobante des microcapsules doit être provoqué non seulement par leurs propriétés chimiques, telles que par exemple l'absence de réactivité à l'égard de la substance à enrober, mais également par leur propension à s'opposer à la formation d'une dispersion stable de fines gouttelettes.

De nombreuses substances peuvent être encapsulées selon le procédé de l'invention qui permet d'enfermer, dans une capsule non réactive à l'égard de cette substance, des matières se présentant sous différents états : liquide, solide, pâteux, etc., telles que les encres, les colorants, les colles, les parfums, les produits phytosanitaires et les produits pharmaceutiques à effet retardé, les réactifs chimiques, les matières odoriférantes, dont on veut empêcher le dégagement des fortes odeurs avant leur emploi, les produits ignifugés, tels les dérivés bromés, libérés par thermofusion des microcapsules, etc.

Comme cela a déjà été exprimé, les substances à encapsuler sont généralement solubilisées dans un solvant organique hydrophobe, ou dans un mélange de solvants organiques hydrophobes, ayant une densité proche de 1, de telle manière que les solutions porteuses de la substance enrobante et de la substance à enrober, puissent être intimement brassées par un fort mouvement d'agitation pour assurer une fine dispersion de l'une et de l'autre solution mises en contact. Mais il est également possible que les substances à encapsuler soient directement introduites dans la solution aqueuse des polymères et/ou copolymères hydrosolubles, sous réserve de n'être pas solubles dans ce milieu et d'être chimiquement inactives à son égard. Dans ce cas, les substances à encapsuler sont très finement divisées et elles sont dans un état solide ou bien sont très finement dispersées sous forme de microscopiques gouttelettes quand elles sont sous forme liquide. Ce peut être le cas, par exemple, dans la micro-encapsulation isodiamétrale d'un solvant organique hydrophobe.

Les solvants organiques hydrophobes utilisés en tant qu'agents de solubilisation de la substance à enrober appartiennent généralement aux groupes des polyaryles, des alkyles polyaryles, etc., tels que par exemple les terphényls partiellement hydrogénés, les mélanges de dibenzylbenzène, les polyalkylbenzènes linéaires de 10 à 14 atomes de carbone en mélange avec du kérosène, les diphényls polyhalogénés tels que le trichlorodiphényl, les alkylnaphtalènes tels que le mono-isopropylnaphtalène, les dérivés du diarylméthane, du triarylméthane, les polyphénols liquides, etc.

Dans le but de réaliser des documents multiples, dits à liasse, dans le domaine des papiers appartenant à la catégorie des papiers autocopiants chimiques autonomes, ou chimiques contacts, la titulaire s'est attachée à exploiter les microcapsules isodiamétrales selon l'invention en développant leur application dans les procédés connus de traitements superficiels des papiers par exemple, du plus simple au plus sophistiqué.

Pour réaliser de tels documents, une première technique employée est celle de l'enduction qui consiste en un dépôt, sur au moins une face du support papier, d'une couche de microcapsules isodiamétrales en une quantité appropriée et une épaisseur régulière.

Pour ce faire, une suspension aqueuse de microcapsules isodiamétrales est placée dans la zone d'alimentation du poste d'enduction. Ladite suspension est formée de microcapsules isodiamétrales, ainsi que de charges, telles que par exemple de l'amidon cru, des fibres de cellulose, et des liants, tels que par exemple de l'amidon cuit, selon une concentration d'environ 40 % en matière sèche.

La suspension de microcapsules est déposée en excès sur le support papier au moyen de divers dispositifs, tels que trempage et prise par rouleau plongeur. Puis elle est ensuite dosée et régulièrement étalée sur le support papier au moyen d'un dispositif approprié qui en élimine l'excédent.

Divers procédés d'enduction ont été pratiqués par la titulaire et sont décrits ci-après à titre d'illustration.

Un premier procédé, appelé Size-press, se classant dans la catégorie des dispositifs à enduction à rouleaux, réalisait le dépôt, le dosage et l'étalement de la suspension en une seule opération dans la zone de serrage des presses. Cette zone de serrage des presses était constituée de deux cylindres horizontaux, l'un étant revêtu d'un matériau dur, tel qu'un caoutchouc de dureté 95° Shore, l'autre étant recouvert d'un matériau moins dur, tel qu'un caoutchouc de dureté 90° Shore. Le support papier à enduire passait entre les deux cylindres, dont l'un au moins était entraîneur, tandis que la suspension des microcapsules isodiamétrales était conduite au-dessus des deux rouleaux, puis placée dans l'espace tangentiel délimité par les surfaces desdits rouleaux. Ainsi, selon cette disposition, le support papier était enduit par la suspension des microcapsules avant de pénétrer dans la zone de serrage des deux cylindres et, de ce fait, la suspension d'enduction était portée instantanément à la vitesse du papier.

Un autre procédé, se classant dans la catégorie des dispositifs à enduction à lame, tels que par exemple à lame d'air ou à barre rotative égalisatrice, réalisait le dépôt, le dosage et l'étalement de la suspension sur le support papier en deux opérations. Dans le cas, par exemple, du dispositif à enduction à barre rotative égalisatrice, la suspension des microcapsules était prélevée par un cylindre enducteur, puis déposée par contact sur le support papier, ledit cylindre pouvant tourner dans le même sens ou dans le sens inverse de la propagation dudit support. Puis le dosage et l'étalement de la couche déposée étaient ensuite assurés au moyen d'un cylindre raclant rotatif, de petit diamètre, tournant dans le sens inverse de la propagation du support enduit.

D'autres procédés d'enduction, bien connus de l'homme de l'art, ont également été expérimentés ou sont susceptibles de l'être avec d'excellents résultats. Procédés entrant dans les catégories précitées, ils portent les noms de Massey, de Reverse Roll, d'offset gravure.

Mais encore, pour réaliser des documents multiples, dits à liasse, à support papier par exemple, par dépôt d'une couche de microcapsules isodiamétrales selon l'invention, une autre technique mise en œuvre par la titulaire est celle de l'impression qui consiste à reporter successivement une même image sur le support, tout en réservant sur ledit support des zones non imprimées appelées réserves.

Divers procédés d'impression — typographie, flexographie, héliogravure et offset — ont été expérimentés par la titulaire, mettant en œuvre une suspension de microcapsules liées, d'une concentration en matière sèche d'environ 40 %, de composition qualitative telle qu'elle a été exprimée antérieurement.

Le procédé d'impression par typographie utilisait un dispositif dont les parties imprimantes en relief étaient de même hauteur et dans le même plan. La suspension des microcapsules isodiamétrales à déposer sur le support papier, placée dans un réceptacle approprié, était prélevée par un premier cylindre qui la transférait par une batterie de cylindres de distribution, jusqu'au cylindre d'impression, muni d'une enveloppe rigide comportant en relief l'image à reproduire sur le support papier, maintenu en contact avec ledit cylindre au moyen d'un rouleau presseur.

Un autre procédé d'impression, dit procédé par flexographie, a également été expérimenté. Le dispositif utilisé comportait un premier cylindre prélevant la suspension des microcapsules isodiamétrales dans un récipient adéquat, qui transférait par contact ladite suspension au cylindre d'impression, muni d'une enveloppe souple comportant en relief l'image à reproduire. Le support papier à traiter était maintenu en contact avec le cylindre d'impression grâce à un autre cylindre, dit cylindre presseur.

Puis le procédé d'impression par héliogravure, expérimenté par la titulaire, a fait usage d'un dispositif comportant un cylindre d'impression, dont la surface est gravée en creux selon la forme de l'image à reproduire. La suspension des microcapsules isodiamétrales placée dans un bac approprié était prélevée par le cylindre gravé, lors de l'immersion partielle, par pénétration dans les alvéoles, tandis que l'excédent de ladite suspension était éliminé par l'intermédiaire d'un racle. Enfin, le support papier à imprimer était mis directement en contact avec le cylindre gravé grâce à un cylindre de contre-pression.

Enfin, la titulaire a également pratiqué, avec les microcapsules isodiamétrales selon l'invention, le procédé d'impression offset. Le dispositif d'impression utilisé comportait un cylindre de prélèvement de la suspension des microcapsules placée dans un réceptacle approprié, puis une batterie de cylindres de distribution transférait la suspension des microcapsules du cylindre de prélèvement au cylindre porteur du cliché. Ensuite, le cylindre appelé porte-blanchet, muni d'une enveloppe caoutchouteuse, assurait le report de l'impression du cylindre porteur du cliché sur le support papier maintenu en contact avec le porte-blanchet au moyen d'un cylindre presseur appelé cylindre de marge.

Toutefois, dans le cas particulier de l'application des microcapsules isodiamétrales selon le procédé offset, la phase aqueuse de la suspension des microcapsules isodiamétrales a été remplacée par au moins un solvant organique, de préférence hydrophile, ou par un mélange de solvants, appartenant par exemple à la famille des glycols, tandis que les parois des microcapsules isodiamétrales avaient été préalablement renforcées. Dès lors, la suspension de microcapsules isodiamétrales utilisées dans le procédé offset l'était dans un solvant ou un mélange de solvants organiques, à une concentration de matière sèche de l'ordre de 40 %.

Dès lors que le support papier a été enduit en continu ou par zones, si nécessaire redressé et enfin séché, il était découpé, liassé et soumis à un test de transfert par dactylographie, permettant de mesurer la qualité du transfert qui s'est révélé excellent jusqu'à la huitième feuille, tandis que les liasses ainsi testées ne présentaient aucun maculage par éclatement accidentel ou prématuré des microcapsules isodiamétrales déposées.

L'invention sera mieux comprise et sa portée plus facilement appréciée grâce aux exemples présentés à titre illustratif.

## Exemple 1

Dans cet exemple, la titulaire a réalisé la production de microcapsules isodiamétrales selon l'invention au moyen d'un micropilote de laboratoire.

Pour ce faire, on a utilisé un réacteur de 1 500 ml du type Prolabo à axe vertical, muni d'une agitation à ancre et d'une turbine Polytron type PTA 10/35 d'un diamètre de 15 mm.

a) Préparation de la solution aqueuse contenant le polymère générateur des parois des microcapsules.

On a introduit successivement dans ce réacteur 430 ml d'eau désionisée et 21,8 g d'une solution de NaOH à 50 %, et le mélange a été porté à la température de 50 °C.

On a alors introduit progressivement, dans la solution alcaline précédente, 28,5 g d'un copolymère d'éthylène/anhydre maléique de masse molaire moyenne d'environ 30 000, en un temps de l'ordre de 5 min, et le mélange ainsi réalisé a été soumis à une agitation lente pendant 30 min, temps nécessaire à la dissolution complète du copolymère précité. A la fin de l'opération de dissolution, on a ajouté au mélange précité 16,8 g de N-butylamine pour élever le pH du milieu à la valeur de 9-10 environ, tout en le maintenant sous agitation lente, c'est-à-dire 30 à 50 tr/min de l'agitateur à ancre.

b) Préparation de la solution organique hydrophobe de la substance à enrober.

Pendant ce temps, on a préparé dans un autre réacteur muni d'une agitation et d'un moyen de chauffage la solution organique hydrophobe contenant la substance à enrober.

Pour cela, on a introduit dans ce deuxième réacteur 286 g de terphényl hydrogéné que l'on a porté à 100 °C sous forte agitation de 100 tr/min, puis on y a ajouté 18,9 g d'un agent chromogène (color-former) à encapsuler, constitué par le mélange de quatre colorants entrant dans la composition à raison des pour-cent en poids ci-après : .

| | |
|---|---|
| Lactone de violet cristallisé (bleu Pergascript I 2R) | 21 % |
| Bleu de benzoylleucométhyle (bleu Pergascript S 4G) | 17 % |
| Rouge de phtalide (rouge Pergascript I 6B) | 11 % |
| Vert de fluorane (olive Pergascript IG) | 51 % |

Tous ces colorants ont été prélevés dans la série Pergascript de la société Ciba-Geigy.

c) Production des microcapsules isodiamétrales.

On a ensuite réalisé le mélange, en dehors de toute agitation, des deux solutions précitées, en introduisant la solution organique hydrophobe de la substance à enrober dans la solution aqueuse du copolymère précité, contenue dans le réacteur Prolabo de 1 500 ml, puis on a réglé la température du mélange à 50 °C.

On a alors soumis pendant 10 min le mélange de ces deux solutions à une action mécanique de cisaillement correspondant à un coefficient de cisaillement de 45 000 s$^{-1}$, en utilisant la turbine Polytron précitée, tout en maintenant le milieu à une température de 50 ± 1 °C.

Après l'écoulement de ces 10 min, au cours duquel se sont formées les microcapsules isodiamétrales, on a arrêté l'action mécanique de cisaillement, et on l'a remplacée par une agitation lente, provoquée par l'agitation à ancre tournant à une vitesse de 30 à 50 tr/min.

Puis on a ajouté, à la suspension des microcapsules isodiamétrales ainsi obtenues, une solution aqueuse d'acide acétique, formée de 26 g dudit acide à 80 % et de 85 g d'eau désionisée, abaissant le pH de la suspension à 6-6,2 pour permettre la réticulation ultérieure des parois des microcapsules.

La suspension ainsi acidifiée a été maintenue sous cette lente agitation et à la température de 50 ± 1 °C pendant 1 1/2 h.

Au bout de ce temps, on a introduit dans la suspension aqueuse des microcapsules isodiamétrales, 68 g d'une solution aqueuse à 45 % d'un précondensat de mélamine/formaldéhyde et, tout en maintenant le milieu sous agitation lente, on en a élevé la température à 75 °C et on l'a maintenue à ce niveau pendant 2 h.

Au bout de ce temps, pendant lequel s'était opérée la réticulation des parois des microcapsules, on a abaissé la température de la suspension des microcapsules réticulées à la température ambiante (20 °C), puis introduit 14 g d'une solution aqueuse d'ammoniaque à 30 % afin d'amener le milieu à un pH de 7.

On a ainsi obtenu une suspension aqueuse de microcapsules ayant une concentration en matière active de 41 %.

Les microcapsules ainsi produites étaient isodiamétrales, car elles avaient un diamètre compris entre 2 et 5 μ pour 80 % d'entre elles et elles n'offraient aucune agglomération, tandis qu'il est courant d'obtenir, par les procédés de l'art antérieur, des capsules dont les diamètres sont distribués entre 5 et 50 μ pour 80 % d'entre elles, avec la présence de nombreux agglomérats.

### Exemple 2

Dans cet exemple, la titulaire a réalisé la fabrication de microcapsules isodiamétrales selon l'invention, au moyen d'une installation industrielle.

Le réacteur utilisé à cet effet avait un diamètre de 1 500 mm, une hauteur de 200 mm, et offrait un volume utile de 2 m$^3$. Il était muni d'un agitateur à ancre, tournant à une vitesse de 12 tr/min et d'une turbine développant un coefficient de cisaillement de 16 000 s$^{-1}$.

a) Préparation de la solution aqueuse contenant le polymère générateur des parois des microcapsules.

On a introduit successivement dans ce réacteur 0,43 m$^3$ d'eau désionisée et 21,8 kg d'une solution de NaOH à 50 % qui ont été portés à 50 ± 0,5 °C. Puis on a introduit 28,5 kg de copolymère d'éthylène et d'anhydride maléique, de masse molaire moyenne 30 000 environ, en un temps de 5 min. Le mélange était alors soumis à une agitation lente pendant 30 min.

A la fin de ce temps, on a ajouté, sous une agitation lente du milieu, 16,8 kg de N-butylamine amenant le pH à la valeur 9-10.

b) Préparation de la solution organique hydrophobe de la substance à enrober.

On a préparé parallèlement, dans un autre réacteur muni d'un agitateur à ancre et d'un moyen de chauffage, la solution organique hydrophobe contenant la substance à enrober, en introduisant dans cet autre réacteur 286 kg de terphényl hydrogéné, que l'on a porté à la température de 100 °C, tout en pratiquant une forte agitation, et en y ajoutant 18,9 kg d'un agent chromogène (color-former) à encapsuler, composé de deux colorants entrant dans la composition à raison des pour-cent en poids suivants :

| | |
|---|---|
| Lactone violet cristallisé | 60 % |
| Bleu benzoyleleucométhylène | 40 % |

Ces colorants appartenaient à la série Pergascript de la société Ciba-Geigy.

c) Production des microcapsules isodiamétrales.

Le mélange des deux solutions précitées a alors été réalisé, en dehors de toute agitation, par introduction dans le réacteur de 2 m$^3$ de la solution organique hydrophobe au sein de la solution aqueuse contenant le copolymère précité. Et la température du mélange était réglée à 50 ± 0,5 °C.

On a alors soumis le mélange des deux solutions, maintenu à cette température, à une action mécanique de cisaillement pendant un temps de 27 min, par usage de la turbine, correspondant à un coefficient de cisaillement de 16 000 s$^{-1}$. Le volume total du mélange des deux solutions précitées subissait 24 recyclages dans la zone de cisaillement.

On a ainsi obtenu, après ce temps de 27 min, une suspension aqueuse de microcapsules, qui était placée sous agitation lente, et à laquelle on a ajouté une solution aqueuse d'acide acétique (26 kg d'acide à 80 % et 85 kg d'eau désionisée), pour amener le pH de la suspension à 6-6,2 et permettre la réticulation ultérieure des parois des microcapsules.

La suspension acidifiée des microcapsules a été maintenue sous agitation lente pendant 1 1/2 h, puis a été traitée par introduction de 68 kg d'une solution aqueuse à 45 % d'un pécondensat de mélamine/formaldéhyde et a été portée à la température de 75 °C, sous agitation lente, et maintenue à cette température pendant 2 h.

Après achèvement de la réticulation des parois des microcapsules, la température de la suspension des microcapsules a été abaissée à la température de 25 °C et l'on a introduit 14 kg d'une solution aqueuse à 30 % d'ammoniaque pour amener le pH du milieu à 7.

On a ainsi obtenu une suspension aqueuse de microcapsules à 41 % de concentration en matière active.

Les microcapsules obtenues étaient isodiamétrales, exemptes de toute agglomération, leur diamètre se situant entre 2 et 5 μ pour 80 % d'entre elles, tandis que 50 % d'entre elles avaient un diamètre moyen de 3,5 μ et ne révélaient aucune agglomération.

Les diamètres des microcapsules isodiamétrales produites selon l'invention ont été déterminés par l'application du principe de Coultner, en utilisant un appareil du type Coultner-Counter Mod TA 11.

## Exemple 3

Cet exemple a la vertu d'illustrer l'influence fondamentale de la température sur le diamètre moyen des microcapsules isodiamétrales, obtenues selon le procédé de l'invention, en permettant d'en régler volontairement cette dimension.

En utilisant le réacteur de 1 500 ml, type Prolabo et en opérant exactement selon les temps, températures, compositions, agitations et actions de cisaillement de 45 000 s$^{-1}$ pratiqués dans l'exemple 1, la titulaire a réalisé six essais de production de microcapsules en soumettant le mélange de la solution aqueuse du copolymère éthylène/anhydride maléique et de la solution de l'agent chromogène dans le terphényl hydrogéné, à l'action de cisaillement précitée, pour des températures variant de 30 à 60 °C au moment de cette action.

Pour chacun de ces essais, le diamètre des microcapsules produites a été exprimé en microns pour 50 % d'entre elles et les résultats obtenus ont été consignés dans le tableau ci-après :

| Numéro de l'essai | Température (°C) pendant l'action de cisaillement | Diamètre (μ) à 50% de capsules |
|---|---|---|
| 1 | 30 | 13,0 |
| 2 | 35 | 9,4 |
| 3 | 40 | 7,0 |
| 4 | 45 | 4,5 |
| 5 | 50 | 3,5 |
| 6 | 60 | 3,0 |

Pour chacun de ces essais, les microcapsules produites selon le procédé de l'invention étaient isodiamétrales. Et l'élévation de la température du milieu au moment de l'action mécanique de cisaillement provoquait la diminution du diamètre moyen desdites microcapsules de 4 à 1 pour une élévation de température de 1 à 2 en pratiquant le même coefficient de cisaillement de 45 000 s$^{-1}$ pendant le même temps de cisaillement fixé à 10 min.

## Exemple 4

Cet exemple illustre la fabrication de matériaux de transfert sur support papier, du type réactif chimique, destinés à faire des duplicatas en liasse de huit exemplaires par le procédé d'enduction à barre rotative égalisatrice.

On a d'abord préparé, dans un récipient muni d'une agitation lente, une suspension liée de microcapsules isodiamétrales produites selon l'exemple 2.

Cette suspension liée comportait :

780 kg de la suspension de microcapsules à 41 % de matières actives,
153 kg d'une solution aqueuse à 17 % en poids d'amidon cuit,
33 kg de fibres de cellulose de diamètre moyen 40 μ,
33 kg, enfin, d'une fine poudre d'amidon cru, de diamètre moyen 15 μ.

Puis ladite suspension liée de microcapsules a été introduite dans la cuve d'alimentation d'un poste d'enduction d'une coucheuse à barre rotative égalisatrice.

La suspension liée des microcapsules isodiamétrales était prélevée par un cylindre enducteur partiellement immergé, de diamètre 250 mm, tournant à une vitesse de 25 tr/min, puis elle était déposée par contact sur le verso d'un support papier, d'un grammage de 52 g/m², dont le recto avait été préalablement enduit d'une charge réactive d'argile acide selon les techniques papetières bien connues de l'homme de l'art.

Le support papier avait 1 100 mm de laize et était animé d'une vitesse de défilement de 200 m/min.

Le dosage et l'étalement de la couche de suspension liée des microcapsules isodiamétrales, déposée sur le verso du support papier, étaient assurés au moyen d'une barre rotative, de diamètre 8 mm, tournant à la vitesse de 10 tr/min dans le sens inverse de la propagation du support papier.

Puis le support papier fraîchement enduit pénétrait dans un séchoir-tunnel dont le gradient de température se répartissait de 100 à 150 °C.

Enfin le support papier séché était redressé et conditionné selon les techniques bien connues de l'homme de l'art.

Dès la fin de ces opérations, on a vérifié que le support papier avait reçu une enduction sur verso de 4 g/m².

Après découpe et constitution de liasses de huit feuillets, on a réalisé un test de transfert par dactylographie, révélant une excellente transmission des caractères jusqu'au huitième feuillet.

Les liasses ainsi testées ne présentaient aucun défaut de maculage par éclatement accidentel ou prématuré des microcapsules isodiamétrales, fabriquées selon le procédé de l'invention.

Exemple 5

Cet exemple illustre la fabrication de matériaux de transfert sur support papier, du type réactif chimique, destinés à faire des duplicatas en liasse de huit exemplaires selon le procédé d'impression par flexographie.

On a utilisé, pour cette application, la même suspension liée de microcapsules isodiamétrales que celle préparée et décrite dans l'exemple 4, qui a été placée dans le réceptacle approprié du poste d'impression par flexographie du dispositif utilisé.

Ce dispositif comportait un premier cylindre de prélèvement de la suspension liée des microcapsules, de diamètre de 200 mm, à trame hélicoïdale, disposant d'un nombre de hachures de 120 par pouce linéaire, de profondeur de taille de 0,055 mm, qui tournait à la vitesse de 470 tr/min. Le cylindre était muni d'un racle évacuant l'excès de la suspension liée des microcapsules.

Ce cylindre de prélèvement transférait, par contact, ladite suspension au cylindre porteur du cliché, muni d'une enveloppe souple comportant en relief l'image à reproduire, de même diamètre que le précédent et tournant à la même vitesse.

Le support papier, d'un grammage de 40 g/m², dont le recto avait été préalablement enduit, par zones, d'une charge réactive d'argile acide, était maintenu, côté verso, en contact avec le cylindre porteur du cliché au moyen d'un cylindre presseur et tournant à la même vitesse que celui-ci.

Le support papier avait 520 mm de laize et défilait à une vitesse de 180 m/min.

A la sortie de la zone d'impression, le support papier passait dans un séchoir à infrarouge, de longueur d'onde de 1 µ, développant une puissance de sèche de 20 kW/h.

Le dosage et l'étalement de la suspension liée des microcapsules isodiamétrales étaient très réguliers et le verso du support papier était revêtu, pour les zones concernées, après impression et séchage, d'une couche représentant 7,5 g/m².

Après découpe et constitution de liasses de seize feuillets, on a réalisé un test de transfert par dactylographie qui a révélé une excellente transmission des caractères jusqu'au seizième feuillet.

Les liasses, après l'exécution de ce test, ne présentaient aucun défaut de maculage par éclatement accidentel ou prématuré des microcapsules isodiamétrales selon l'invention.

Exemple 6

Cet exemple illustre la fabrication de matériaux de transfert sur support papier du type réactif chimique, destiné à faire des duplicatas en liasses de huit exemplaires selon le procédé d'impression offset.

Pour cette application, on a utilisé la même suspension aqueuse des microcapsules isodiamétrales que celle produite dans l'exemple 1. Toutefois, la phase aqueuse de cette suspension a été remplacée par du monopropylèneglycol, tandis que les parois des microcapsules isodiamétrales étaient renforcées par le même copolymère ayant servi à l'encapsulation.

Dès lors, la suspension des microcapsules isodiamétrales utilisée dans le procédé offset avait la composition suivante en pour-cent en poids :

| | |
|---|---|
| Microcapsules isodiamétrales en sec | 45 % |
| Solvant : monopropylèneglycol | 55 % |

Le dispositif offset utilisé comportait un cylindre de prélèvement de la suspension des microcapsules, placée dans un réceptacle approprié, puis une batterie de cylindres de distribution transférait la suspension des microcapsules isodiamétrales du cylindre de prélèvement au cylindre porte-cliché, enfin un cylindre dit porte-blanchet, muni d'une enveloppe de caoutchouc, assurait le report de l'impression du cliché sur le support papier.

Enfin, le support papier était maintenu en contact avec le cylindre porte-blanchet grâce au cylindre presseur appelé cylindre de marge.

Les cylindres porte-cliché, porte-blanchet et de marge avaient un diamètre de 22 pouces et étaient animés d'une vitesse de rotation de 100 tr/min.

Le support papier, d'un grammage de 52 g/m², dont le recto avait été préalablement enduit, par zones, d'une charge réactive d'argile acide, était maintenu côté verso en contact avec le cylindre porte-blanchet.

Le support papier avait une laize de 520 mm et défilait à une vitesse de 180 m/min.

A la sortie de la zone d'impression, le support papier passait dans un séchoir à infrarouge, de longueur d'onde de 1 μ, développant une puissance de sèche de 20 kW/h.

Le dosage et l'étalement de la suspension des microcapsules isodiamétrales étaient très réguliers et le verso du support papier revêtu, pour les zones concernées, après impression et séchage, d'une couche de 4 g/m².

Après découpe et constitution des liasses de huit feuillets, on a réalisé le même test de transfert par dactylographie que dans les exemples 4 et 5. Ce test a révélé une très bonne transmission des caractères, même sur le huitième feuillet.

Les liasses, après exécution de ce test, ne présentaient aucun défaut de maculage, par éclatement accidentel ou prématuré des microcapsules isodiamétrales selon l'invention.

## Revendications

1. Procédé de micro-encapsulation de substances diverses dans le but d'obtenir des microcapsules simultanément isodiamétrales et de diamètre réglé, qui consiste

à mettre en présence une solution aqueuse de polymères et/ou copolymères hydrosolubles comportant au moins l'une des fonctions chimiques appartenant au groupe constitué par les radicaux —COOH, —OH, —CONH₂, —CONHR, —CONRR', —NH₂, —NHR et NRR', constituant la matière de l'enveloppe-réceptacle de la capsule, et une solution des substances à enrober dans au moins un solvant organique hydrophobe, de densité proche de 1, du groupe des solvants polyaryles,

à amener le milieu liquide ainsi constitué à une température choisie entre 15 et 85 °C, de préférence entre 35 et 60 °C,

à le soumettre pendant un temps compris dans l'intervalle de 1 à 60 min, de préférence de 20 à 35 min à une action mécanique de cisaillement correspondant à un coefficient de cisaillement compris entre 8 000 et 70 000 s⁻¹, de préférence compris entre 15 000 et 45 000 s⁻¹ en le maintenant à cette température choisie pendant l'action de cisaillement,

à régler le pH de la suspension des microcapsules isodiamétrales dans le milieu aqueux résultant de cette action dans l'intervalle de 3,5 à 7,5,

à introduire un agent de réticulation dans la suspension précitée, et

à abaisser la température de la suspension précitée de préférence jusqu'à la température ambiante.

2. Procédé de micro-encapsulation selon la revendication 1, caractérisé en ce que le volume total du milieu liquide constitué par le solvant porteur de la matière enrobante et le solvant porteur de la substance à enrober est soumis à un nombre de recyclages dans la zone de cisaillement compris entre 10 et 200, mais de préférence entre 12 et 55.

3. Procédé de micro-encapsulation selon la revendication 1, caractérisé en ce que la solution aqueuse des polymères et/ou copolymères est préparée par dissolution à chaud de ces matières à une température comprise entre 10 et 100 °C, mais de préférence entre 30 et 60 °C.

4. Procédé de micro-encapsulation selon la revendication 1, caractérisé en ce que la solution de substances à enrober dans au moins un solvant organique hydrophobe est obtenue par dissolution à chaud de ladite substance, à une température comprise entre 10 et 150 °C, et de préférence entre 80 et 110 °C.

5. Procédé de micro-encapsulation selon la revendication 1, caractérisé en ce que la solution aqueuse des polymères et/ou copolymères est à une concentration en pour-cent en poids de matière sèche comprise entre 12 et 15 %, mais de préférence entre 3 et 8 %.

6. Procédé de micro-encapsulation selon la revendication 1, caractérisé en ce que la concentration en pour-cent en poids de la solution de substance à enrober dans au moins un solvant organique hydrophobe est comprise entre 20 et 2 %, mais de préférence entre 10 et 5 %, et est de 100 % quand la substance à enrober est le solvant organique hydrophobe lui-même.

7. Procédé de micro-encapsulation selon la revendication 1, caractérisé en ce que la solution aqueuse contenant les polymères et/ou copolymères, mise en présence de la solution organique hydrophobe contenant la substance à enrober, l'est en une quantité telle que le rapport massique des polymères et/ou copolymères hydrosolubles et de la substance à enrober soit compris entre 0,05 et 0,3 et de préférence entre 0,07 et 0,15.

8. Procédé de micro-encapsulation selon la revendication 1, caractérisé en ce que les polymères et/ou copolymères hydrosolubles destinés à former l'enveloppe des microcapsules sont choisis dans ce groupe constitué par les polymères et/ou copolymères dérivés de la cellulose, les protéines et leurs dérivés, les polymères et/ou copolymères à base de mélamine et de formaldéhyde et d'urée et de formaldéhyde, les polyacides, les polyesters, les copolymères d'anhydrides, les polyacrylamides et les copolymères d'acrylamide.

**0 059 949**

Claims

1. Process of microencapsulating various substances with the aim of obtaining microcapsules which are simultaneously isodiametric and of predetermined diameter, which consists of putting in each other's presence an aqueous solution of hydrosoluble polymers and/or copolymers comprising at least one of the chemical functions belonging to the group constituted by the radicals —COOH, —OH, —CONH$_2$, —CONHR, —CONRR', —NH$_2$, —NHR, and —NRR, constituting the material of the casing receptacle of the capsule, and a solution of the substances to be encased in at least one hydrophobic organic solvent, of a density close to 1, of the group of polyaryl solvents,

of bringing the liquid medium thus constituted to a temperature selected between 15 and 85 °C and preferably between 35 and 60 °C,

of subjecting it during a period of time comprised within the interval of 1 to 60 min, but preferably from 20 to 35 min to a mechanical shearing action corresponding to a shearing coefficient of between 8 000 and 70 000 s$^{-1}$, preferably of between 15 000 and 45 000 s$^{-1}$ and maintaining the selected temperature during shearing,

of adjusting the pH of the thus obtained suspension of the isodiametric microcapsules to a range from 3,5 to 7,5,

of introducing into said suspension a cross-linking agent and

of lowering the temperature of said suspension preferably to room temperature.

2. Process of microencapsulating according to Claim 1, characterized in that the total volume of liquid medium, constituted by the solvent carrying the encasing material and the solvent carrying the substance to be encased, is subjected to a number of recycling operations in the shearing zone comprising between 10 and 200, but preferably between 12 and 55.

3. Process of microencapsulating according to Claim 1, characterized in that the aqueous solution of polymers and/or copolymers is prepared by dissolving these materials under heat at a temperature comprised between 10 and 100 °C, but preferably between 30 and 60 °C.

4. Process of microencapsulating according to Claim 1, characterized in that the solution of substances to be encased in at least one hydrophobic organic solvent is obtained by dissolving the said substance under heat at a temperature comprised between 10 and 150 °C, and preferably between 80 and 110 °C.

5. Process of microencapsulating according to Claim 1, characterized in that the aqueous solution of polymers and/or copolymers is at a concentration in percent by weight of dry matter of between 12 and 15 % but preferably between 3 and 8 %.

6. Process of microencapsulating according to Claim 1, characterized in that the concentration in percent by weight of the solution of the substance to be encased in at least one hydrophobic organic solvent is between 20 and 2 %, but preferably between 10 and 5 %, and is at 100 % when the substance to be encased is the hydrophobic organic solvent itself.

7. Process of microencapsulating according to Claim 1, characterized in that the aqueous solution, containing the polymers and/or copolymers brought into the presence of the hydrophobic organic solution containing the substance to be encased, is in a quantity such that the mass ratio of the hydrosoluble polymers and/or copolymers to the substance to be encased is between 0.05 and 0.3 and preferably between 0.07 and 0.15.

8. Process of microencapsulating according to Claim 1, characterized in that the hydrosoluble polymers and/or copolymers intended to form the casing of the microcapsules are selected from within the group constituted by the polymers and/or copolymers derived from cellulose, the proteins and their derivatives, the polymers and/or copolymers based on melamine and formaldehyde and on urea and formaldehyde, the polyacids, the polyesters, the copolymers of anhydrides, the polyacrylamides and the copolymers of acrylamide.

Patentansprüche

1. Verfahren zur Mikroeinkapselung von verschiedenen Substanzen in isodiametrischen Mikrokapseln mit eingestelltem Durchmesser, bei dem man

eine wässrige Lösung von wasserlöslichen Polymeren und/oder Copolymeren, die wenigstens eine chemische Funktion der von den Resten —COOH, —OH, —CONH$_2$, —CONHR, —CONRR', —NH$_2$, —NHR und —NRR' gebildeten Gruppe haben, und die das umhüllende und aufnehmende Material der Kapsel bilden, mit einer Lösung der zu umhüllenden Substanzen in wenigstens einem organischen hydrophoben Lösungsmittel einer Dichte nahe bei 1 aus der Gruppe der Polyaryllösungsmittel in Berührung bringt,

das so zusammengesetzte flüssige Milieu auf eine Temperatur zwischen 15 und 85, vorzugsweise zwischen 35 und 60 °C bringt,

während eines Zeitraums von 1 bis 60, vorzugsweise 20 bis 35 min der mechanischen Einwirkung von Scherkräften unterwirft, die einem Scherkoeffizienten zwischen 8 000 und 70 000, vorzugsweise 15 000 und 45 000 s$^{-1}$ entsprechen, und während der Einwirkung der Scherkräfte bei der gewählten Temperatur hält,

14

nach der mechanischen Einwirkung der Scherkräfte das pH der dadurch erhaltenen Suspension der isodiametrischen Mikrokapseln auf den Bereich von 3,5 bis 7,5 einstellt,

in die vorgenannte Suspension ein Vernetzungsmittel einführt und

die Temperatur der genannten Suspension vorzugsweise auf Raumtemperatur senkt.

2. Verfahren zur Mikroeinkapselung nach Anspruch 1, dadurch gekennzeichnet, dass das Gesamtvolumen des flüssigen Milieus aus dem die Umhülungssubstanz enthaltenden Lösungsmittel und dem die zu umhüllende Substanz enthaltenden Lösungsmittel 20 bis 200, vorzugsweise 12 bis 55 Kreisläufen in der Scherzone unterworfen wird.

3. Verfahren zur Mikroeinkapselung nach Anspruch 1, dadurch gekennzeichnet, dass die wasserige Lösung der Polymeren und/oder Copolymeren durch Auflösung der Substanzen in der Wärme bei einer Temperatur zwischen 10 und 100, vorzugsweise zwischen 30 und 60 °C, hergestellt wird.

4. Verfahren zur Mikroeinkapselung nach Anspruch 1, dadurch gekennzeichnet, dass die Lösung der zu umhüllenden Substanzen in wenigstens einem hydrophoben organischen Lösungsmittel durch Auflösen der genannten Substanz in der Wärme bei einer Temperatur zwischen 10 und 150, vorzugsweise zwischen 80 und 110 °C, hergestellt wird.

5. Verfahren zur Mikroeinkapselung nach Anspruch 1, dadurch gekennzeichnet, dass die wässerige Lösung der Polymeren und/oder Copolymeren eine Konzentration, berechnet in Prozent der Trockensubstanz, zwischen 12 und 15, vorzugsweise 3 und 8 %, besitzt.

6. Verfahren zur Mikroeinkapselung nach Anspruch 1, dadurch gekennzeichnet, dass die Konzentration in Gewichtsprozent der Lösung der zu umhüllenden Substanz in wenigstens einem hydrophoben organischen Lösungsmittel zwischen 20 und 2, vorzugsweise zwischen 10 und 5 %, beträgt und dass sie 100 % beträgt, wenn die zu umhüllende Substanz das hydrophobe organische Lösungsmittel selbst ist.

7. Verfahren zur Mikroeinkapselung nach Anspruch 1, dadurch gekennzeichnet, dass die die Polymeren und/oder Copolymeren enthaltende wässerige Lösung, die mit der die zu umhüllende Substanz enthaltenden organischen hydrophoben Lösung in Berührung gebracht wird, darin in einer solchen Menge vorliegt, dass das Massenverhältnis der wasserlöslichen Polymeren und/oder Copolymeren und der zu umhüllenden Substanz zwischen 0,05 und 0,3, vorzugsweise zwischen 0,07 und 0,15, beträgt.

8. Verfahren zur Mikroeinkapselung nach Anspruch 1, dadurch gekennzeichnet, dass die wasserlöslichen Polymeren und/oder Copolymeren, die die Hülle der Mikrokapseln bilden sollen, aus der Gruppe der polymeren und/oder copolymeren Cellulosederivate, der Proteine oder ihrer Derivate, der Polymeren und/oder Copolymeren auf der Grundlage von Melaminformaldehyd und Harnstoffformaldehyd, den Polysäuren, den Polyestern, den Anhydridcopolymeren, den Polyacrylamiden und den Acrylamidcopolymeren gewählt sind.